# EUROPEAN PATENT SPECIFICATION

(11) **EP 4 630 407 B1**
(45) Date of publication and mention of the grant of the patent: **22.07.2026**
(21) Application number: 23837857.4
(22) Date of filing: 01.12.2023
(51) Int. Cl.: C07D 213/75, C07C 233/02, C07C 233/16, C07C 233/58, C07C 233/60, C07C 233/88, A23L 27/20

(54) **2-METHYL-2-(1-METHYLCYCLOHEXYL)PROPANAMIDE DERIVATIVES AND 1-(TERT-BUTYL)-CYCLOHEXANE-1-CARBOXAMIDE DERIVATIVES FOR USE IN UMAMI FLAVOR COMPOSITIONS**
2-METHYL-2-(1-METHYLCYCLOHEXYL)PROPANAMID-DERIVATE UND 1-(TERT-BUTYL)-CYCLOHEXAN-1-CARBOXAMID-DERIVATE ZUR VERWENDUNG IN UMAMI-AROMA-ZUSAMMENSETZUNGEN
DÉRIVÉS DE 2-MÉTHYL-2-(1-MÉTHYLCYCLOHEXYL)PROPANAMIDE ET DÉRIVÉS DE 1-(TERT-BUTYL)-CYCLOHEXANE-1-CARBOXAMIDE DESTINÉS À ÊTRE UTILISÉS DANS DES COMPOSITIONS D'ARÔME UMAMI

(30) Priority: 05.12.2022 US 202263386005 P
(43) Date of publication of application: 15.10.2025
(73) Proprietor: International Flavors & Fragrances, Inc., New York, NY 10019 (US)
(72) Inventor: KAZIMIERSKI, Arkadiusz, UNION BEACH, New Jersey 07735 (US); NIEDEVELD, Cornelis, UNION BEACH, New Jersey 07735 (US); RENNIE, Laura Ashley, UNION BEACH, New Jersey 07735 (US); SCHNEIDER, Erica, UNION BEACH, New Jersey 07735 (US); SHARAFBAFI, Negin, UNION BEACH, New Jersey 07735 (US); MONTELEONE, Michael G., UNION BEACH, New Jersey 07735 (US)
(74) Representative: Mewburn Ellis LLP
(86) International application number: PCT/US2023/082020
(87) International publication number: WO 2024/123607

(56) References cited:
- WO-A1-2008/046895
- US-A1- 2009 110 796
- US-A1- 2011 070 329
- DANIEL K. YARBROUGH: "COMPOSITIONS INCORPORATING AN UMAMI FLAVOR AGENT", IP.COM, 8 December 2016 (2016-12-08), WEST HENRIETTA, NY, US, pages 1 - 127, XP013173665, ISSN: 1533-0001

## Description

### Field of the Invention

The present invention relates to novel flavor compounds and their use in providing and enhancing umami taste in flavor compositions.

### Background of the Invention

Food industry has made significant effort to modify and harmonize taste in food products. The basic categories of taste include salty, sweet, sour, bitter and umami. The discovery of novel flavor compounds that modify these basic tastes enables the creation of desirable flavors.

Those with skill in the art appreciate how small differences in molecular structures can result in significant differences in compound notes and flavor properties. These distinctive properties can be highly valuable as they provide unique and distinguished characters to flavor compositions. However, many of these distinctive properties can also be undesirable and, thus, would render molecules not suited for flavor use. Thus, those skilled in the art would recognize that the discovery and development of a novel compound suitable for flavor use is unpredictable.

Daniel K. Yarbrough: "Compositions incorporating an umami flavor agent", IP.com, 8 December 2016 (2016-12-08), pages 1-127, XP013173665, West Henrietta, NY, US; ISSN: 1533-0001 describes compositions comprising an umami flavor agent.

US 2009/0110796 A1 describes aromatic neomenthylamides as a flavoring substance or flavoring substance mixture.

WO 2008/046895 A1 describes substituted bicyclo[4.1.0]heptane-7-carboxylic acid amides and derivatives thereof as food flavor substances.

US 2011/0070329 A1 describes aromatic 1-tert-butylcyclohexanecarboxamide derivatives as cooling agents.

### Summary of the Invention

The present invention provides a compound as defined in claim 1, a flavor composition as defined in claim 3, a method of providing or enhancing an umami taste as defined in claim 10, a flavor product as defined in claim 11, a use as defined in claim 12, a use as defined in claim 13, and a use as defined in claim 14.

The present invention provides novel chemicals, and their use to enhance the flavor of foodstuff, chewing gums, dental and oral hygiene products, medicinal products and the like. In one embodiment, the present invention relates to a novel flavor compound, 2-methyl-2-(1-methylcyclohexyl)propanamide, represented by Formula A set forth below: 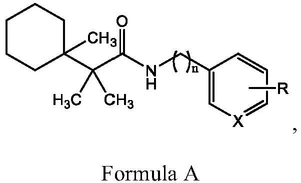
wherein n represents an integer of 0-4, and preferably 0-2;
R represents a substituent in any position of the benzene ring and is independently selected from the group consisting of hydrogen, a C₁-C₄ alkyl group and -OR', wherein R' is a C₁-C₄ alkyl group; and
X represents C or N.

Another embodiment of the present invention relates to a flavor composition comprising the compound of Formula A.

Another embodiment of the present invention relates to a flavor composition comprising the compound of Formula A and a 1-tert-butylcyclohexane carboxamide represented by Formula B set forth below: 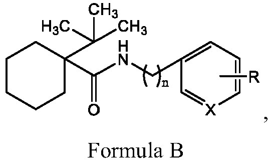 wherein n, R and X are defined as above.

Another embodiment of the present invention relates to a flavor composition comprising corresponding compounds of Formula A and Formula B, wherein the compound of Formula A is present in an amount of about 1 to about 50% by weight and the compound of Formula B is present in an amount of about 50 to about 99% by weight.

Another embodiment of the present invention relates to the flavor compositions provided above further comprising an umami compound.

Another embodiment of the present invention relates to a method of providing or enhancing an umami taste in an other flavor composition comprising the step of adding the flavor compositions provided above to the other flavor composition.

Another embodiment of the present invention relates to a flavor product comprising the flavor compositions provided above.

These and other embodiments of the present invention will be apparent by reading the following specification.

### Detailed Description of the Invention

The present invention relates to novel compounds represented by Formula A and Formula B set forth above and their advantageous use in providing an umami taste in flavor compositions. Further, the present invention relates to a flavor composition comprising a mixture of corresponding compounds of Formula A and Formula B.

The present invention relates to, for example, but not limited to, the following structures and flavor compositions.

### N-(3,4-Dimethylphenyl)-2-methyl-2-(1-methylcyclohexyl)propanamide (Structure A1)

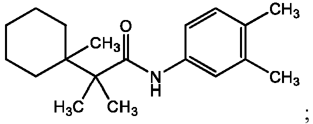

A flavor composition comprising Structure A1 and *1-(tert-butyl)-N-(3,4-*dimethylphenyl)cyclohexane-1-carboxamide (Structure B1):

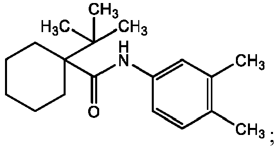

### 2-Methyl-2-(1-methylcyclohexyl)-N-(3,4,5-trimethylphenyl)propanamide (Structure A2):

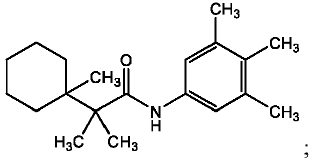

A flavor composition comprising Structure A2 and 1-(tert-butyl)-N-(3,4,5-trimethylphenyl)cyclohexane-1-carboxamide (Structure B2):

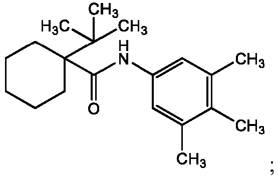

### 2-Methyl-2-(1-methylcyclohexyl)-N-phenethylpropanamide (Structure A3):

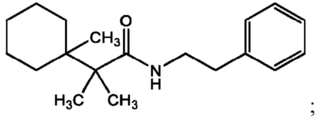

A flavor composition comprising Structure A3 and *1-(tert-butyl)-N-*phenethylcyclohexane-1-carboxamide (Structure B3):

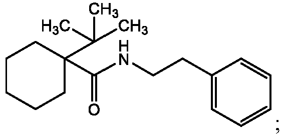

### N-(4-Methoxyphenyl)-2-methyl-2-(1-methylcyclohexyl)propanamide (Structure A4):

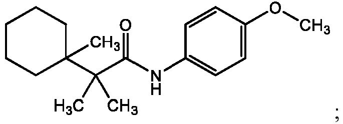

A flavor composition comprising Structure A4 and *1-(tert-butyl)-N-(4-*methoxyphenyl)cyclohexane-1-carboxamide (Structure B4):

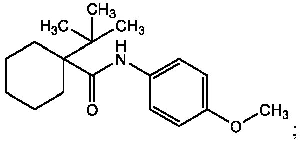

### N-(4-Ethoxyphenyl)-2-methyl-2-(1-methylcyclohexyl)propanamide (Structure A5):

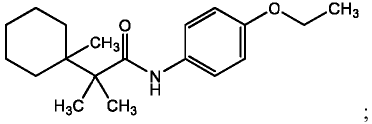

A flavor composition comprising Structure A5 and *1-(tert-butyl)-N-(4-*ethoxyphenyl)cyclohexane-1-carboxamide (Structure B5):

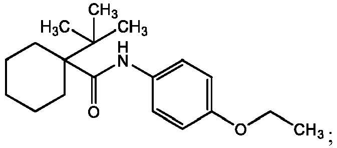

### N-(4-Isopropoxyphenyl)-2-methyl-2-(1-methylcyclohexyl)propanamide (Structure A6)

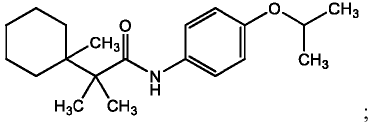

A flavor composition comprising Structure A6 and *1-(tert-butyl)-N-(4-*isopropoxyphenyl)cyclohexane-1-carboxamide (Structure B6):

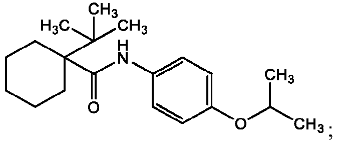

### N-(2,4-Dimethoxyphenyl)-2-methyl-2-(1-methylcyclohexyl)propanamide (Structure A7):

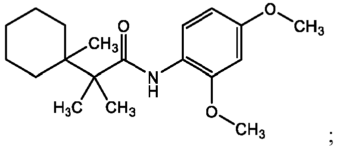

A flavor composition comprising Structure A7 and *1-(tert-butyl)-N-(2,4-*dimethoxyphenyl)cyclohexane-1-carboxamide (Structure B7):

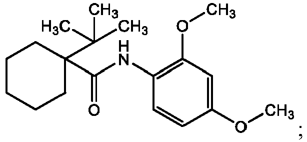

### N-(4-Methoxyphenethyl)-2-methyl-2-(1-methylcyclohexyl)propanamide (Structure A8):

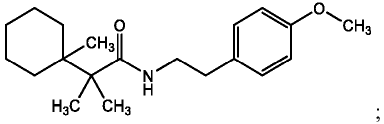

A flavor composition comprising Structure A8 and *1-(tert-butyl)-N-(4-*methoxyphenethyl)cyclohexane-1-carboxamide (Structure B8):

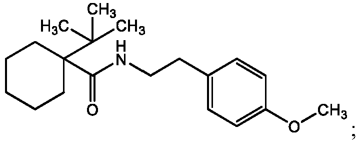

### 2-Methyl-2-(1-methylcyclohexyl)-N-(6-methylpyridin-3-yl)propanamide (Structure A9):

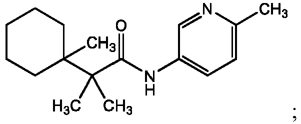

A flavor composition comprising Structure A9 and 1-(tert-butyl)-N-(6-methylpyridin-3-yl)cyclohexane-1-carboxamide (Structure B9):

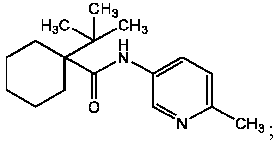

### N-(6-Methoxypyridin-3-yl)-2-methyl-2-(1-methylcyclohexyl)propanamide (Structure A10):

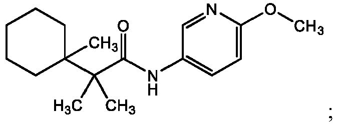

A flavor composition comprising Structure A10 and 1-(tert-butyl)-N-(6-methoxypyridin-3-yl)cyclohexane-1-carboxamide (Structure B10):

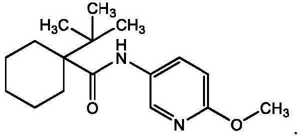

Those with skill in the art will recognize that the compounds of the present invention may contain chiral centers, thereby providing a number of stereoisomers of the claimed compounds. It is intended herein that the compounds of the present invention include isomeric mixtures as well as individual isomers that may be separated using techniques known to those having skill in the art. Suitable techniques include chromatography such as high performance liquid chromatography, referred to as HPLC, particularly silica gel chromatography, and gas chromatography trapping known as GC trapping. Yet, commercial versions of such products are mostly offered as mixtures.

### 2-Methyl-2-(1-methylcyclohexyl)propanamides of Formula A can be prepared via a general scheme depicted as follows:

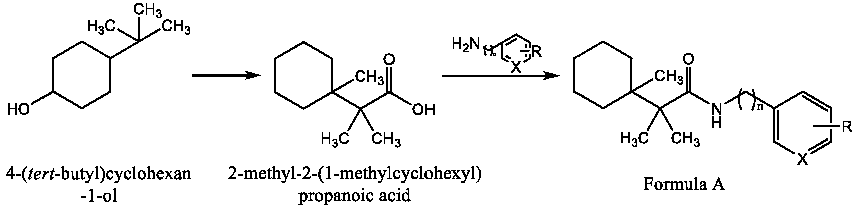

### 1-tert-Butylcyclohexane carboxamides of Formula B can be prepared via a general scheme depicted as follows:

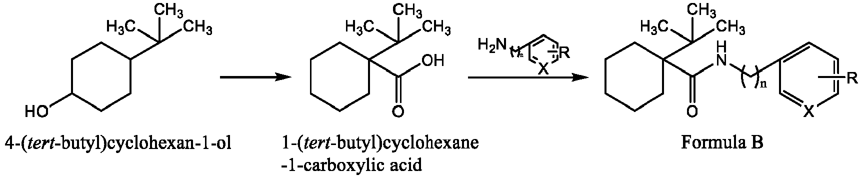

The present invention has identified a novel flavor compound, a 2-methyl-2-(1-methylcyclohexyl)propanamide of Formula A. Further, the present invention has made surprising and unexpected discovery of the synergistic flavor effect achieved by combining a 2-methyl-2-(1-methylcyclohexyl)propanamide of Formula A and a corresponding 1-tert-butylcyclohexane carboxamide of Formula B.

Compounds of Formula A and Formula B can each be used alone or in combinations thereof. In particular, a 2-methyl-2-(1-methylcyclohexyl)propanamide of Formula A can be used in combination with a corresponding 1-tert-butylcyclohexane carboxamide of Formula B. The compounds of the present invention can be used in further combination with additional flavor compositions, solvents, adjuvants and the like.

In one embodiment, a flavor composition of the present invention contains a 2-methyl-2-(1-methylcyclohexyl)propanamide of Formula A in an amount of about 1 to about 50% by weight and a corresponding 1-tert-butylcyclohexane carboxamide of Formula B in an amount of about 50 to about 99% by weight. In a preferred embodiment, the flavor composition contains a 2-methyl-2-(1-methylcyclohexyl)propanamide of Formula A in an amount of from about 5 to about 50% by weight and a corresponding 1-tert-butylcyclohexane carboxamide of Formula B in an amount of from about 50 to about 95% by weight. In a more preferred embodiment, the flavor composition contains a 2-methyl-2-(1-methylcyclohexyl)propanamide of Formula A in an amount of from about 10 to about 50% by weight and a corresponding 1-tert-butylcyclohexane carboxamide of Formula B in an amount of from about 50 to about 90% by weight.

Umami compounds may include, for example, but not limited to, glutamate, ribonucleotides and salts thereof. Glutamate includes, for example, but not limited to, monosodium glutamate (MSG), potassium glutamate and calcium glutamate. Ribonucleotides such as 5'-ribonucleotides include, for example, but not limited to, guanosine 5'-monophosphate (GMP), inosine 5'-monophosphate (IMP) and adenosine 5'-monophosphate (AMP).

In one embodiment, a flavor composition of the present invention contains the compounds of the present invention and an umami compound. In an embodiment, the flavor composition contains a 2-methyl-2-(1-methylcyclohexyl)propanamide of Formula A and an umami compound. In a particular embodiment, the flavor composition contains a 2-methyl-2-(1-methylcyclohexyl)propanamide of Formula A, a corresponding 1-tert-butylcyclohexane carboxamide of Formula B and an umami compound such as monosodium glutamate (MSG) (CAS No. 142-47-2), 5'-ribonucleotide sodium (CAS No. 80702-47-2) or a mixture thereof.

When the compounds of the present invention are used in a flavoring composition, they can be combined with other conventional flavoring materials or adjuvants, which are well known in the art and have been extensively described in the past. Conventional flavoring materials include saturated fatty acids, unsaturated fatty acids, amino acids; alcohols including primary and secondary alcohols; esters; carbonyl compounds including ketones; aldehydes; lactones; cyclic organic materials including benzene derivatives, acyclic compounds, heterocyclies such as furans, pyridines, pyrazines and the like; sulfur-containing compounds including thiols, sulfides, disulfides and the like; proteins; lipids; carbohydrates; so-called flavor potentiators such as monosodium glutamate; magnesium glutamate, calcium glutamate, guanylates and inosinates; natural flavoring materials such as hydrolyzates, cocoa, vanilla and caramel; essential oils and extracts such as anise oil, clove oil and the like; and artificial flavoring materials such as vanillin, ethyl vanillin and the like. Requirements for adjuvants include: (1) that they be non-reactive with the compounds of the present invention; (2) that they be organoleptically compatible with the compounds of the present invention, whereby the flavor of the ultimate consumable product to which the compounds of the present invention are added is not detrimentally affected by the use of the adjuvants; and (3) that they be ingestible acceptable and thus nontoxic or otherwise non-deleterious. In addition, other flavor materials, vehicles, stabilizers, thickeners, surface active agents, conditioners and flavor intensifiers can also be included.

The term "foodstuff" as used herein includes both solid and liquid ingestible materials for man or animals, which materials usually do, but need not, have nutritional value. Thus, foodstuffs include meats, gravies, soups, convenience foods, malt, alcoholic and other beverages, milk and dairy products, seafood, including fish, crustaceans, mollusks and the like, candies, vegetables, cereals, soft drinks, snacks, dog and cat foods, other veterinary products and the like.

The terms "flavor composition" and "flavor formulation" mean the same and refer to a consumer composition that produces a pleasant or desired flavor. The flavor composition contains a compound or a mixture of compounds. The flavor composition of the present invention is a consumer composition comprising a compound of Formula A of the present invention.

The term "flavor product" means a consumer product containing a compound of the present invention and further a foodstuff, a chewing gum, a dental product, an oral hygiene product or a medicinal product.

As used herein, the term "a" or "an" is understood to mean one or more. The term "a compound" is understood to mean one or more of the compounds represented by Formula A, Formula B or a mixture thereof as described herein. The term "a substituent" is understood to mean one or more substitutes in Formula A or Formula B as described herein. The term "corresponding" is understood to mean a compound of Formula A and a compound of Formula B, wherein n, R and X in the compound of Formula A are each identical to those in the compound of Formula B,

The terms "synergistic flavor effect" or "synergistic umami" mean the same and refer to the interaction between two or more components or chemicals when the combined umami effect is enhanced and larger than the sum of the umami effect of the individual components. In one embodiment, the synergistic flavor effect of the present invention refers to the synergistic umami effect of a 2-methyl-2-(1-methylcyclohexyl)propanamide of Formula A and an umami compound. In another embodiment, the synergistic flavor effect of the present invention refers to the synergistic umami effect of a 2-methyl-2-(1-methylcyclohexyl)propanamide of Formula A and a corresponding 1-tert-butylcyclohexane carboxamide of Formula B. In a preferred embodiment, the synergistic flavor effect of the present invention refers to the synergistic umami effect of a 2-methyl-2-(1-methylcyclohexyl)propanamide of Formula A, a corresponding *1-tert-*butylcyclohexane carboxamide of Formula B and an umami compound. In a more preferred embodiment, the synergistic flavor effect of the present invention refers to the synergistic umami effect of a 2-methyl-2-(1-methylcyclohexyl)propanamide of Formula A, a corresponding *1-tert-*butylcyclohexane carboxamide of Formula B and an umami compound of monosodium glutamate (MSG) (CAS No. 142-47-2), 5'-ribonucleotide sodium (CAS No. 80702-47-2) or a mixture thereof.

The term "olfactory acceptable amount" is understood to mean the amount of a compound in a flavor composition, wherein the compound will contribute its individual olfactory characteristics. However, the olfactory effect of the flavor composition will be the sum of effect of each of the flavor ingredients. Thus, the compound of the present invention can be used to improve or enhance the aroma characteristics of the flavor composition, or by modifying the olfactory reaction contributed by other ingredients in the composition. The olfactory acceptable amount may vary depending on many factors including other ingredients, their relative amounts and the olfactory effect that is desired.

Generally, the olfactory acceptable amount of the compounds of the present invention employed in a flavor composition is greater than about 1 part per billion by weight, preferably from about 1 part per billion to about 500 parts per million by weight, more preferably from about 10 part per billion to about 100 parts per million by weight, even more preferably from about 100 parts per billion to about 20 parts per million by weight, and further more preferably from about 200 parts per billion to 20 parts per million by weight. Those with skill in the art will be able to employ the desired amount to provide desired flavor effect and intensity. In addition to the compounds of the present invention, other materials can also be used in conjunction with the flavor composition to encapsulate and/or deliver the flavor. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

Some preferred polymers include polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde), or a combination thereof.

The following are provided as specific embodiments of the present invention. As used herein all percentages are weight percent unless otherwise noted, ppb is understood to stand for parts per billion, ppm is understood to stand for parts per million, L is understood to be liter, mL is understood to be milliliter, Kg is understood to be kilogram, g is understood to be gram, mol is understood to be mole, mmol is understood to be millimole, and M is understood to be molar. IFF as used in the examples is understood to mean International Flavors & Fragrances Inc., New York, NY, USA.

### EXAMPLE I

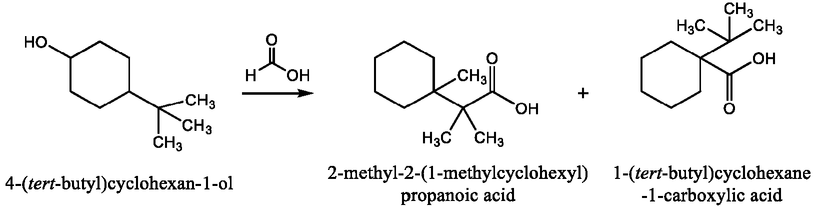

### Preparation of 2-Methyl-2-(1-methylcyclohexyl)propanoic Acid and 1-(tert-Butyl)cyclohexane-1-carboxylic Acid:

2-Methyl-2-(1-methylcyclohexyl)propanoic acid and 1-(tert-butyl)cyclohexane-1-carboxylic acid having a weight ratio of 3:7 were prepared according to a known method (Recueil des Travaux Chimiques des Pays-Bas (1970), 89 (5): 521-534).

2-Methyl-2-(1-methylcyclohexyl)propanoic Acid:
¹H NMR (400 MHz, CDCl₃) δ: 12.00 (br s, 1H), 1.16 (s, 6H), 0.99 (s, 3H), 0.90-1.80 (m, 10H)
1-(tert-Butyl)cyclohexane-1-carboxylic Acid:
   ¹H NMR (400 MHz, CDCl₃) δ: 11.00 (br s, 1H), 2.12 (m, 2H), 1.66 (m, 3H), 1.00-1.40 (m, 5H), 0.96 (s, 9H)

### EXAMPLE II

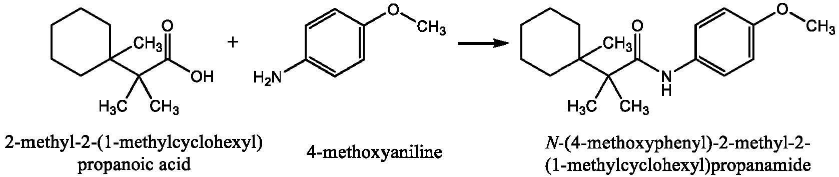

### Preparation of N-(4-Methoxyphenyl)-2-methyl-2-(1-methylcyclohexyl)propanamide (Structure A4):

4-Methoxyaniline (1.6 g) and 2-chloro-1-methylpyridin-1-ium iodide (3.3 g) were added to a dichloromethane (DCM) solution of the 2-methyl-2-(1-methylcyclohexyl)propanoic acid and 1-(tert-butyl)cyclohexane-1-carboxylic acid mixture (2 g) (obtained in EXAMPLE I). The reaction mixture was heated at reflux for 1 hour and allowed to cool down to ambient temperature. Triethylamine (N(CH₂CH₃)₃) (2.3 g) was added to the reaction mixture, which was refluxed for additional 20 hours. Workup was subsequently conducted by adding hydrochloric acid (HCl) followed by extraction with DCM. The column chromatographic separation was performed using the hexane/ethyl acetate solvent system with a solvent gradient of ethyl acetate from 0 to 15%. *N-*(4-Methoxyphenyl)-2-methyl-2-(1-methylcyclohexyl)propanamide was identified and obtained as white solid with a yield of 10%.
¹H NMR (400 MHz, CDCl₃) δ: 7.37-7.48 (m, 2H), 7.20 (br s, 1H), 6.82-6.93 (m, 2H), 3.81 (s, 3H), 1.37-1.72 (m, 9H), 1.27 (s, 6H), 1.05-1.16 (m, 1H), 1.04 (s, 3H)

### Example III

A series of water solutions of N-(4-methoxyphenyl)-2-methyl-2-(1-methylcyclohexyl)propanamide (Structure A4) were prepared and evaluated for flavor. The flavor profile is reported in the following:

| **Structure A4** | **Flavor Profile** |
|---|---|
| 100 ppb | Very slight bitter, minimal cooling, drying linger, minimal umami |
| 200 ppb | Slight cooling, slight bitter, drying, very slight umami |
| 375 ppb | Slight bitter, cooling, umami, linger |
| 500 ppb | Clear and moderate umami, slight bitter, cooling, linger |
| 750 ppb | Clear and moderate umami, cooling, slightly soapy, slightly waxy |
| 1 ppm | Strong umami, cooling, tingly, tongue scratching |
| 2 ppm | Strong and lingering umami, good mouthfeel, cooling, tingling |
| 5 ppm | Strong and lingering umami, moderate and lingering cooling, lingering bitter, drying |
| 10 ppm | Moderate umami, moderate cooling, lingering bitter, tingling, brackish linger |
| 15 ppm | Moderate umami, moderate cooling, lingering bitter, creamy, mineral, drying |

Structure A4 exhibited umami at all above concentrations.

### Example IV

A monosodium glutamate (MSG) solution (5000 ppm) was prepared in water. The flavor profile of the MSG solution with added Structure A4 is reported in the following:

| **Structure A4** | **Umami Strength** |
|---|---|
| 0 | MSG base umami |
| 100 ppb | Added effect of Structure A4 and MSG |
| 200 ppb | Synergistic umami of Structure A4 and MSG, lingering umami |
| 375 ppb | Synergistic umami of Structure A4 and MSG, lingering umami |
| 500 ppb | Synergistic umami of Structure A4 and MSG, lingering umami |
| 750 ppb | Synergistic umami of Structure A4 and MSG, lingering umami |
| 1 ppm | Synergistic umami of Structure A4 and MSG, lingering umami |
| 2 ppm | Synergistic umami of Structure A4 and MSG, lingering umami |
| 5 ppm | Synergistic umami of Structure A4 and MSG, strong cooling |
| 10 ppm | Synergistic umami of Structure A4 and MSG, strong cooling |
| 15 ppm | Synergistic umami of Structure A4 and MSG, strong cooling |

As used herein, the term "synergistic umami" refers to the umami effect of the Structure A4 and MSG combination that is stronger than the added umami effect of individual Structure A4 and MSG. Synergistic umami was achieved in combinations of Structure A4 ranging from 200 ppb to 15 ppm and MSG (5000 ppm).

### Example V

Similarly, synergistic umami was also achieved when Structure A4 (2 ppm) was added in a Ribotide^{®} solution (1000 ppm).

### EXAMPLE VI

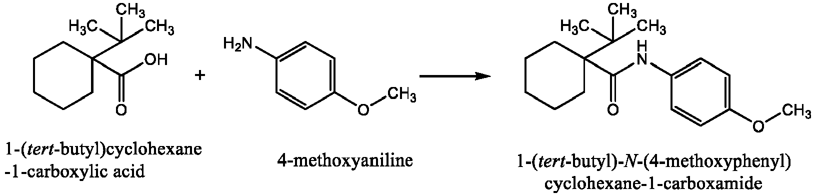

### Preparation of 1-(tert-Butyl)-N-(4-methoxyphenyl)cyclohexane-1-carboxamide (Structure B4):

4-Methoxyaniline (1.6 g) and 2-chloro-1-methylpyridin-1-ium iodide (3.3 g) were added to a dichloromethane (DCM) solution of the 2-methyl-2-(1-methylcyclohexyl)propanoic acid and 1-(tert-butyl)cyclohexane-1-carboxylic acid mixture (2 g, obtained in EXAMPLE I). The reaction mixture was heated at reflux for 1 hour and allowed to cool to ambient temperature. Triethylamine (N(CH₂CH₃)₃) (2.3 g) was added to the reaction mixture, which was refluxed for additional 20 hours. Workup was subsequently conducted by adding hydrochloric acid (HCl) followed by extraction with DCM. The column chromatographic separation was performed using the hexane/ethyl acetate solvent system with a solvent gradient of ethyl acetate from 0 to 15%. 1-(tert-Butyl)-*N*-(4-methoxyphenyl)cyclohexane-1-carboxamide was identified and obtained as white solid with a yield of 10%.
¹H NMR (500 MHz, CDCl₃) δ: 7.42 (m, 2H), 7.18 (br s, 1H), 6.87 (m, 2H), 3.79 (s, 3H), 2.14 (m, 2H), 1.69 (m, 3H), 1.10-1.40 (m, 5H), 0.99 (s, 9H).

Structure B4 in water (375 ppb) provided slight umami, slight sweetness and bitter linger.

### EXAMPLE VII

### Preparation of N-(4-Methoxyphenyl)-2-methyl-2-(1-methylcyclohexyl)propanamide (Structure A4) and 1-(tert-Butyl)-N-(4-methoxyphenyl)cyclohexane-1-carboxamide (Structure B4) Mixture:

The mixture of 2-methyl-2-(1-methylcyclohexyl)propanoic acid and 1-(*tert*-butyl)cyclohexane-1-carboxylic acid (1 g, obtained in EXAMPLE I) was heated with thionyl chloride (SOCl₂) (0.97 g) for 1 hour. The excess of SOCl₂ was evaporated. The obtained chloride intermediate was added dropwise as dichloromethane (DCM) solution to a mixture of 4-methoxyaniline (0.8 g) and triethylamine ((C₂H₅)₃N) (1.26 g). Reaction was mixed overnight and then poured into hydrochloric acid (HCl) solution (1 N) (27 mL). The resulted mixture was extracted with DCM solution three times. The organic layers were combined, washed with water and brine, and dried over sodium sulfate (Na₂SO₄). Chromatographic purification was applied to provide the mixture of *N*-(4-methoxyphenyl)-2-methyl-2-(1-methylcyclohexyl)propanamide and 1-(tert-butyl)-*N*-(4-methoxyphenyl)cyclohexane-1-carboxamide with a weight ratio of 3 to 7.
¹H NMR (500 MHz, CDCl₃) δ: 7.42 (m, 2H), 7.18 (br s, 1H), 6.87 (m, 2H), 3.79 (s, 3H), 2.14 (m, 2H), 1.69 (m, 3H), 1.10-1.40 (m, 5H), 0.99 (s, 9H)

The obtained mixture in water (200 ppb) provided mild umami flavor with tingling and mild lingering characters.

### EXAMPLE VIII

### Preparation and Evaluation of N-(4-Methoayphenyl)-2-methyl-2-(1-methylcyclohexyl)propanamide (Structure A4) and 1-(tert-Butyl)-N-(4-methoxyphenyl)cyclohexane-1-carboxamide (Structure B4) Mixtures of Different Ratios:

Mixtures of N-(4-methoxyphenyl)-2-methyl-2-(1-methylcyclohexyl)propanamide (Structure A4) and 1-(tert-butyl)-N-(4-methoxyphenyl)cyclohexane-1-carboxamide (Structure B4) were prepared by mixing two compounds at different ratios. "Ratio" refers to the weight ratio of Structure A4 and Structure B4. Water solutions of the mixtures (375 ppb) were prepared and evaluated for flavor in water, MSG solution (5000 ppm) and a salt solution (containing NaCl of 3750 ppm, Ribotide^{®} of 35 ppm and MSG of 840 ppm), respectively. Water, MSG solution and the salt solution were each used as base controls.

| **Ratio** | **Water** | **MSG** | **Salt Solution** |
|---|---|---|---|
| Base | Blank taste | Slight bitter linger, some tingling, slight umami | Slight bitter linger, slight umami |
| 10:90 | Slight bitter, slight cooling, slight sweet, some umami | Slight bitter linger, some tingling, more umami | More umami slowly built up and linger, slight bitter linger |
| 25:75 | Slight bitter, slight cooling, slight sweet, clear synergistic umami, mouthfeel | Brothy, clear synergistic umami | Brothy, synergistic umami with a clean character, noticeable onset, strong peak and linger, some bitter linger |
| 50:50 | Slight bitter, slight cooling, slight sweet and linger, synergistic umami and creamy | Slightly metallic, synergistic umami, some astringency and drying | Brothy, synergistic umami with delayed onset and linger, mouthfeel and some tingling |

The Structure A4 and B4 mixture of 50:50 ratio (4 ppm) was further evaluated in Ribotide^{®} (1000 ppm). Synergistic umami was also achieved.

Combinations of Structure A4 and B4 and umami compounds provided synergistic umami. The effect of the Structure A4 and B4 mixture of a 25:75 ratio was particularly strong and desirable.

In all evaluations, *N*-(4-methoxyphenyl)-2-methyl-2-(1-methylcyclohexyl)propanamide (Structure A4) provided upfront and lingering umami character and achieved synergistic umami when combined with Structure B4 and/or an additional umami compound.

### EXAMPLE IX

The following Formula A and Formula B compounds were similarly prepared according to the procedures described in the above.

### N-(3,4-Dimethylphenyl)-2-methyl-2-(1-methylcyclohexyl)propanamide (Structure A1) and 1-(tert-Butyl)-N-(3,4-dimethylphenyl)cyclohexane-1-carboxamide (Structure B1):

A mixture of *N*-(3,4-dimethylphenyl)-2-methyl-2-(1-methylcyclohexyl)propanamide and *1-(tert-Butyl)-N-(3,4-*dimethylphenyl)cyclohexane-1-carboxamide having a weight ratio of 3:7 was prepared.
¹H NMR (500 MHz, CDCl₃) δ: 7.37 (d, J=2.0 Hz, 1H), 7.22 (dd, J=8.0, 2.0 Hz, 1H), 7.19 (br s, 1H), 7.08 (d, J=8.0 Hz, 1H), 2.26 (s, 3H), 2.22 (s, 3H), 2.10-2.19 (m, 2H), 1.62-1.78 (m, 3H), 1.32-1.48 (m, 4H), 1.08-1.21 (m, 1H), 0.99 (s, 9H)

The obtained product exhibited mild umami flavor at 375 ppb in water.

### 2-Methyl-2-(1-methylcyclohexyl)-N-(3,4,5-trimethylphenyl)propanamide (Structure A2) and 1-(tert-Butyl)-N-(3,4,5-trimethylphenyl)cyclohexane-1-carboxamide (Structure B2):

**A** mixture of 2-methyl-2-(1-methylcyclohexyl)-N-(3,4,5-trimethylphenyl)propanamide and *1-(tert-*butyl)-*N*-(3,4,5-trimethylphenyl)cyclohexane-1-carboxamide having a weight ratio of 3:7 was prepared.
¹H NMR (400 MHz, CDCl₃) δ: 7.23 (s, 2H), 7.17 (br s, 1H), 2.30 (s, 6H), 2.11-2.20 (m, 5H), 1.63-1.81 (m, 3H), 1.33-1.52 (m, 4H), 1.10-1.25 (m, 1H), 1.01 (s, 9H)

The obtained product exhibited mild umami flavor at 375 ppb in water.

### 2-Methyl-2-(1-methylcyclohexyl)-N-phenethylpropanamide (Structure A3) and 1-(tert-Butyl)-N-phenethylcyclohexane-1-carboxamide (Structure B3):

A mixture of 2-methyl-2-(1-methylcyclohexyl)-*N*-phenethylpropanamide (Structure A3) and *1-(tert-butyl)-N-*phenethylcyclohexane-1-carboxamide having a weight ratio of 3:7 was prepared.
¹H NMR (500 MHz, CDCl₃) δ: 7.20-7.35 (m, 5H), 5.68 (br s, 1H), 3.59 (m, 2H), 2.87 (t, J=7.00 Hz, 2H), 1.94 (m, 2H), 1.56 (m, 3H), 1.00-1.30 (m, 5H), 0.86 (s, 9H)

The obtained product exhibited mild umami flavor at 375 ppb in water.

### N-(4-Ethoxyphenyl)-2-methyl-2-(1-methylcyclohexyl)propanamide (Structure A5) and 1-(tert-Butyl)-N-(4-ethoxyphenyl)cyclohexane-1-carboxamide (Structure B5):

A mixture of *N-*(4-ethoxyphenyl)-2-methyl-2-(1-methylcyclohexyl)propanamide and *1-(tert-butyl)-N-(4-*ethoxyphenyl)cyclohexane-1-carboxamide having a weight ratio of (3:7) was prepared.

¹H NMR (500 MHz, CDCl₃) δ: 7.40 (m, 2H), 7.18 (br s, 1H), 6.86 (m, 2H), 4.01 (q, J=6.95 Hz, 2H), 2.14 (m, 2H), 1.72 (m, 3H), 1.40 (t, J=6.95 Hz, 3H), 1.10-1.50 (m, 5H), 0.99 (s, 9H)

The obtained product exhibited mild umami flavor at 375 ppb in water.

### N-(4-Isopropoxyphenyl)-2-methyl-2-(1-methylcyclohexyl)propanamide (Structure A6) and 1-(tert-Butyl)-N-(4-isopropoxyphenyl)cyclohexane-1-carboxamide (Structure B6):

A mixture of *N*-(4-isopropoxyphenyl)-2-methyl-2-(1-methylcyclohexyl)propanamide and *1-(tert-butyl)-N-*(4-isopropoxyphenyl)cyclohexane-1-carboxamide having a weight ratio of 3:7 was prepared.

¹H NMR (500 MHz, CDCl₃) δ: 7.40 (m, 2H), 7.17 (br s, 1H), 6.86 (m, 2H), 4.49 (sept, J=6.00 Hz, 1H), 2.15 (m, 2H), 1.68 (m, 3H), 1.32 (d, J=6.00 Hz, 6H), 1.10-1.40 (m, 5H), 0.99 (s, 9H)

The obtained product exhibited mild umami flavor at 375 ppb in water.

### N-(2,4-Dimethoxyphenyl)-2-methyl-2-(1-methylcyclohexyl)propanamide (Structure A7) and 1-(tert-Butyl)-N-(2,4-dimethoxyphenyl)cyclohexane-1-carboxamide (Structure B7):

A mixture of *N*-(2,4-dimethoxyphenyl)-2-methyl-2-(1-methylcyclohexyl)propanamide and *1-(tert-*butyl)-*N*-(2,4-dimethoxyphenyl)cyclohexane-1-carboxamide having a weight ratio of 3:7 was prepared.
¹H NMR (400 MHz, CDCl₃) δ: 8.27-8.34 (m, 1H), 7.87 (br s, 1H), 6.45-6.53 (m, 2H), 3.87 (s, 3H), 3.80 (s, 3H), 2.11-2.24 (m, 2H), 1.56-1.81 (m, 3H), 1.30-1.52 (m, 4H), 1.07-1.21 (m, 1H), 1.00 (s, 9H)

The obtained product exhibited mild umami flavor at 375 ppb in water.

### N-(4-Methoxyphenethyl)-2-methyl-2-(1-methylcyclohexyl)propanamide (Structure A8) and 1-(tert-Butyl)-N-(4-methoxyphenethyl)cyclohexane-1-carboxamide (Structure B8):

A mixture of *N*-(4-methoxyphenethyl)-2-methyl-2-(1-methylcyclohexyl)propanamide and 1-(*tert*-butyl)-N-(4-methoxyphenethyl)cyclohexane-1-carboxamide having a weight ratio of 3:7 was prepared.
¹H NMR (500 MHz, CDCl₃) δ: 7.14 (m, 2H), 6.84 (m, 2H), 5.59 (br s, 1H), 3.79 (s, 3H), 3.55 (m, 2H), 2.80 (t, J=7.00 Hz, 2H), 1.94 (m, 2H), 1.57 (m, 3H), 1.00-1.30 (m, 5H), 0.86 (s, 9H)

The obtained product exhibited mild umami flavor at 375 ppb in water.

### 2-Methyl-2-(1-methylcyclohexyl)-N-(6-methylpyridin-3-yl)propanamide (Structure A9) and 1-(tert-Butyl)-N-(6-methylpyridin-3-yl)cyclohexane-1-carboxamide (Structure B9):

A mixture of 2-methyl-2-(1-methylcyclohexyl)-*N*-(6-methylpyridin-3-yl)propanamide and *1-(tert-*butyl)-*N*-(6-methylpyridin-3-yl)cyclohexane-1-carboxamide having a weight ratio of 3:7 was prepared using 6-methylpyridin-3-amine.
¹H NMR (400 MHz, CDCl₃) δ: 8.63 (m, 1H), 8.31 (m, 1H), 7.71 (br s, 1H), 7.22 (apparent d, J=8.40 Hz, 1H), 2.60 (s, 3H), 2.25 (m, 2H), 1.73 (m, 3H), 1.10-1.50 (m, 5H), 1.01 (s, 9H)

The obtained product exhibited mild umami flavor at 375 ppb in water.

### N-(6-Methoxypyridin-3-yl)-2-methyl-2-(1-methylcyclohexyl)propanamide (Structure A10) and 1-(tert-Butyl)-N-(6-methoxypyridin-3-yl)cyclohexane-1-carboxamide: (Structure B10):

A mixture of *N*-(6-methoxypyridin-3-yl)-2-methyl-2-(1-methylcyclohexyl)propanamide and 1-(*tert*-butyl)-*N*-(6-methoxypyridin-3-yl)cyclohexane-1-carboxamide having a weight ratio of 3:7 was prepared using 6-methoxypyridin-3-amine.
¹H NMR (400 MHz, CDCl₃) δ: 8.13 (d, J=2.7 Hz, 1H), 7.96 (dd, J=8.9, 2.7 Hz, 1H), 7.25 (br s, 1H), 6.75 (d, J=8.9 Hz, 1H), 3.93 (s, 3H), 2.12-2.25 (m, 2H), 1.62-1.82 (m, 3H), 1.31-1.51 (m, 4H), 1.10-1.25 (m, 1H), 1.01 (s, 9H)

The obtained product exhibited mild umami flavor at 375 ppb in water.

Among all the above products, the Structure A4 and B4 mixture provided the strongest and most desirable umami taste.

## Claims

1. A compound of Formula A: 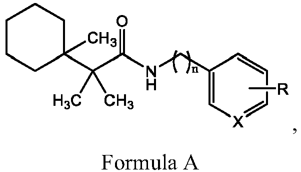
wherein n represents an integer of 0-4;
R represents a substituent in any position of the benzene ring and is independently selected from the group consisting of hydrogen, a C₁-C₄ alkyl group and -OR', wherein R^{'} is a C₁-C₄ alkyl group; and
X represents C or N.

2. The compound of claim 1 selected from the group consisting of:
*N*-(3,4-dimethylphenyl)-2-methyl-2-(1-methylcyclohexyl)propanamide;
2-methyl-2-(1-methylcyclohexyl)-*N*-(3,4,5-trimethylphenyl)propanamide;
2-methyl-2-(1-methylcyclohexyl)-*N*-phenethylpropanamide;
*N*-(4-methoxyphenyl)-2-methyl-2-(1-methylcyclohexyl)propanamide;
*N*-(4-ethoxyphenyl)-2-methyl-2-(1-methylcyclohexyl)propanamide;
*_{N}*-(4-isopropoxyphenyl)-2-methyl-2-(1-methylcyclohexyl)propanamide;
*N*-(2,4-dimethoxyphenyl)-2-methyl-2-(1-methylcyclohexyl)propanamide;
*N*-(4-methoxyphenethyl)-2-methyl-2-(1-methylcyclohexyl)propanamide;
2-methyl-2-(1-methylcyclohexyl)-*N*-(6-methylpyridin-3-yl)propanamide;
*N*-(6-methoxypyridin-3-yl)-2-methyl-2-(1-methylcyclohexyl)propanamide; and
a mixture thereof.

3. A flavor composition comprising an olfactory acceptable amount of a compound of claim 1 or 2.

4. The flavor composition of claim 3 comprising an olfactory acceptable amount of corresponding compounds of Formula A and Formula B: 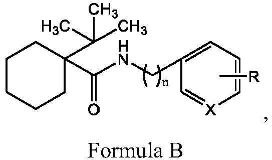
wherein n represents an integer of 0-4;
R represents a substituent in any position of the benzene ring and is independently selected from the group consisting of hydrogen, a C₁-C₄ alkyl group and -OR^{'}, wherein R^{'} is a C₁-C₄ alkyl group; and
X represents C or N.

5. The flavor composition of claim 4, wherein the corresponding compounds of Formula A and Formula B are selected from the group consisting of:
N-(3,4-dimethylphenyl)-2-methyl-2-(1-methylcyclohexyl)propanamide and 1-(tert-butyl)-*N*-(3,4-dimethylphenyl)cyclohexane-1-carboxamide;
2-methyl-2-(1-methylcyclohexyl)-*N*-(3,4,5-trimethylphenyl)propanamide and *1-(tert-*butyl)-*N*-(3,4,5-trimethylphenyl)cyclohexane-1-carboxamide;
2-methyl-2-(1-methylcyclohexyl)-*N*-phenethylpropanamide and *1-(tert-butyl)-N-*phenethylcyclohexane-1-carboxamide;
*N*-(4-methoxyphenyl)-2-methyl-2-(1-methylcyclohexyl)propanamide and 1-(*tert*-butyl)-*N*-(4-methoxyphenyl)cyclohexane-1-carboxamide;
*N*-(4-ethoxyphenyl)-2-methyl-2-(1-methylcyclohexyl)propanamide and *1-(tert-butyl)-N-*(4-ethoxyphenyl)cyclohexane-1-carboxamide;
*N*-(4-isopropoxyphenyl)-2-methyl-2-(1-methylcyclohexyl)propanamide and *1-(tert-*butyl)-*N*-(4-isopropoxyphenyl)cyclohexane-1-carboxamide;
*N*-(2,4-dimethoxyphenyl)-2-methyl-2-(1-methylcyclohexyl)propanamide and *1-(tert-*butyl)-*N*-(2,4-dimethoxyphenyl)cyclohexane-1-carboxamide;
*N*-(4-methoxyphenethyl)-2-methyl-2-(1-methylcyclohexyl)propanamide and *1-(tert-*butyl)-N-(4-methoxyphenethyl)cyclohexane-1-carboxamide;
2-methyl-2-(1-methylcyclohexyl)-N-(6-methylpyridin-3-yl)propanamide and *1-(tert-*Butyl)-*N*-(6-methylpyridin-3-yl)cyclohexane-1-carboxamide; and
*N*-(6-methoxypyridin-3-yl)-2-methyl-2-(1-methylcyclohexyl)propanamide and *1-(tert-*butyl)-*N*-(6-methoxypyridin-3-yl)cyclohexane-1-carboxamide.

6. The flavor composition of claim 5, wherein the corresponding compounds of Formula A and Formula _{B} are N-(4-methoxyphenyl)-2-methyl-2-(1-methylcyclohexyl)propanamide and 1-(*tert*-but_{y}l)-*N*-(4-methoxyphenyl)cyclohexane-1-carboxamide.

7. The flavor composition of claim 4, wherein the olfactory acceptable amount is about 1ppb or greater, and wherein the compound of Formula A is present in an amount of about 1 to 50% by weight and the compound of Formula B is present in an amount of about 50% to about 99% by weight.

8. The flavor composition of claim 3 or 4 further comprising an umami compound.

9. The flavor composition of claim 8, wherein the umami compound is selected from the group consisting of monosodium glutamate; 5'-ribonucleotide sodium; and a mixture thereof.

10. A method of providing or enhancing an umami taste of a first flavor composition comprising the step of adding to the first flavor composition a second flavor composition, wherein the second flavor composition is a flavor composition according to any one of claims 3 to 9.

11. A flavor product comprising the flavor composition of any one of claims 3 to 9.

12. Use of a compound according to claim 1 or 2 to provide or enhance an umami taste of a flavor composition.

13. Use of a compound according to claim 1 or 2 to improve or enhance aroma characteristics of a flavor composition.

14. Use of a compound according to claim 1 or 2 or a flavor composition according to any one of 3 to 9 in a flavor product.

## Patentansprüche

1. Verbindung der Formel A: 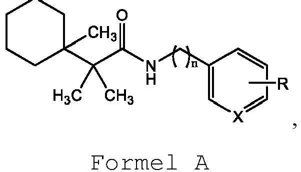
wobei n für eine ganze Zahl von 0-4 steht;
R für einen Substituenten in einer beliebigen Position des Benzolrings steht und unabhängig aus der Gruppe bestehend aus Wasserstoff, einer C₁-C₄-Alkylgruppe und - OR' ausgewählt ist, wobei R' eine C₁-C₄-Alkylgruppe ist; und
X für C oder N steht.

2. Verbindung nach Anspruch 1, ausgewählt aus der Gruppe bestehend aus:
N-(3,4-Dimethylphenyl)-2-methyl-2-(1-methylcyclohexyl)propanamid;
2-Methyl-2-(1-methylcyclohexyl)-N-(3,4,5-trimethylphenyl)propanamid;
2-Methyl-2-(1-methylcyclohexyl)-N-phenethylpropanamid;
N-(4-Methoxyphenyl)-2-methyl-2-(1-methylcyclohexyl)propanamid;
N-(4-Ethoxyphenyl)-2-methyl-2-(1-methylcyclohexyl)propanamid;
N-(4-Isopropoxyphenyl)-2-methyl-2-(1-methylcyclohexyl)propanamid;
N-(2,4-Dimethoxyphenyl)-2-methyl-2-(1-methylcyclohexyl)propanamid;
N-(4-Methoxyphenethyl)-2-methyl-2-(1-methylcyclohexyl)propanamid;
2-Methyl-2-(1-methylcyclohexyl)-N-(6-methylpyridin-3-yl)propanamid;
N-(6-Methoxypyridin-3-yl)-2-methyl-2-(1-methylcyclohexyl)propanamid und
einem Gemisch davon.

3. Geschmacksstoffzusammensetzung, umfassend eine olfaktorisch annehmbare Menge einer Verbindung nach Anspruch 1 oder 2.

4. Geschmacksstoffzusammensetzung nach Anspruch 3, umfassend eine olfaktorisch annehmbare Menge entsprechender Verbindungen der Formel A und Formel B: 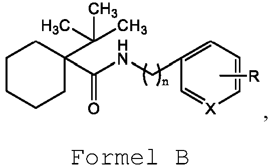
wobei n für eine ganze Zahl von 0-4 steht;
R für einen Substituenten in einer beliebigen Position des Benzolrings steht und unabhängig aus der Gruppe bestehend aus Wasserstoff, einer C₁-C₄-Alkylgruppe und - OR' ausgewählt ist, wobei R' eine C₁-C₄-Alkylgruppe ist; und
X für C oder N steht.

5. Aromazusammensetzung nach Anspruch 4, wobei die entsprechenden Verbindungen der Formel A und Formel B ausgewählt sind aus der Gruppe bestehend aus:
N-(3,4-Dimethylphenyl)-2-methyl-2-(1-methylcyclohexyl)propanamid und 1-(tert-Butyl)-N-(3,4-dimethylphenyl)cyclohexan-1-carboxamid;
2-Methyl-2-(1-methylcyclohexyl)-N-(3,4,5-trimethylphenyl)propanamid und 1-(tert-Butyl)-N-(3,4,5-trimethylphenyl)cyclohexan-1-carboxamid;
2-Methyl-2-(1-methylcyclohexyl)-N-phenethylpropanamid und 1-(tert-Butyl)-N-phenethylcyclohexan-1-carboxamid; N-(4-Methoxyphenyl)-2-methyl-2-(1-methylcyclohexyl)propanamid und 1-(tert-Butyl)-N-(4-methoxyphenyl)cyclohexan-1-carboxamid; N-(4-Ethoxyphenyl)-2-methyl-2-(1-methylcyclohexyl)propanamid und 1-(tert-Butyl)-N-(4-ethoxyphenyl)cyclohexan-1-carboxamid; N-(4-Isopropoxyphenyl)-2-methyl-2-(1-methylcyclohexyl)propanamid und 1-(tert-Butyl)-N-(4-isopropoxyphenyl)cyclohexan-1-carboxamid; N-(2,4-Dimethoxyphenyl)-2-methyl-2-(1-methylcyclohexyl)propanamid und 1-(tert-Butyl)-N-(2,4-dimethoxyphenyl)cyclohexan-1-carboxamid; N-(4-Methoxyphenethyl)-2-methyl-2-(1-methylcyclohexyl)propanamid und 1-(tert-Butyl)-N-(4-methoxyphenethyl)cyclohexan-1-carboxamid;
2-Methyl-2-(1-methylcyclohexyl)-N-(6-methylpyridin-3-yl)propanamid und 1-(tert-Butyl)-N-(6-methylpyridin-3-yl)cyclohexan-1-carboxamid und
N-(6-Methoxypyridin-3-yl)-2-methyl-2-(1-methylcyclohexyl)propanamid und 1-(tert-Butyl)-N-(6-methoxypyridin-3-yl)cyclohexan-1-carboxamid.

6. Geschmacksstoffzusammensetzung nach Anspruch 5, wobei es sich bei den entsprechenden Verbindungen der Formel A und Formel B um N-(4-Methoxyphenyl)-2-methyl-2-(1-methylcyclohexyl)propanamid und 1-(tert-Butyl)-N-(4-methoxyphenyl)cyclohexan-1-carboxamid handelt.

7. Geschmacksstoffzusammensetzung nach Anspruch 4, wobei die olfaktorisch annehmbare Menge etwa 1 ppb oder mehr beträgt und wobei die Verbindung der Formel A in einer Menge von etwa 1 bis 50 Gew.-% vorliegt und die Verbindung der Formel B in einer Menge von etwa 50 bis etwa 99 Gew.-% vorliegt.

8. Geschmacksstoffzusammensetzung nach Anspruch 3 oder 4, die ferner eine Umami-Verbindung umfasst.

9. Geschmacksstoffzusammensetzung nach Anspruch 8, wobei die Umami-Verbindung aus der Gruppe bestehend aus Mononatriumglutamat; 5'-Ribonukleotid-Natrium; und einer Mischung davon ausgewählt ist.

10. Verfahren zum Bereitstellen oder Verstärken eines Umami-Geschmacks einer ersten Geschmacksstoffzusammensetzung, umfassend den Schritt des Zugebens einer zweiten Geschmacksstoffzusammensetzung zu der ersten Geschmacksstoffzusammensetzung, wobei es sich bei der zweiten Geschmacksstoffzusammensetzung um eine Geschmacksstoffzusammensetzung nach einem der Ansprüche 3 bis 9 handelt.

11. Geschmacksstoffprodukt, umfassend die Geschmacksstoffzusammensetzung nach einem der Ansprüche 3 bis 9.

12. Verwendung einer Verbindung nach Anspruch 1 oder 2 zur Bereitstellung oder Verstärkung eines Umami-Geschmacks einer Geschmacksstoffzusammensetzung.

13. Verwendung einer Verbindung nach Anspruch 1 oder 2 zur Verbesserung oder Verstärkung der Aromaeigenschaften einer Geschmacksstoffzusammensetzung.

14. Verwendung einer Verbindung nach Anspruch 1 oder 2 oder einer Geschmacksstoffzusammensetzung nach einem der Ansprüche 3 bis 9 in einem Geschmacksstoffprodukt.

## Revendications

1. Composé de formule A : 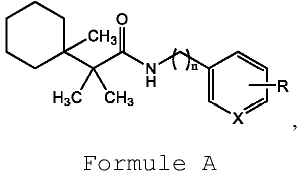
dans laquelle n représente un entier de 0-4 ;
R représente un substituant à une position quelconque du cycle benzénique et est indépendamment choisi dans le groupe constitué par hydrogène, un groupe alkyle en C₁-C₄ et -OR', où R' est un groupe alkyle en C₁-C₄ ; et
X représente C ou N.

2. Composé selon la revendication 1 choisi dans le groupe constitué par :
N-(3,4-diméthylphényl)-2-méthyl-2-(1-méthylcyclohexyl)propanamide ;
2-méthyl-2-(1-methylcyclohexyl)-N-(3,4,5-triméthylphényl)propanamide ;
2-méthyl-2-(1-méthylcyclohexyl)-N-phénéthylpropanamide ;
N-(4-méthoxyphényl)-2-méthyl-2-(1-méthylcyclohexyl)propanamide ;
N-(4-éthoxyphényl)-2-méthyl-2-(1-méthylcyclohexyl)propanamide ;
N-(4-isopropoxyphényl)-2-méthyl-2-(1-méthylcyclohexyl)propanamide ;
N-(2,4-diméthoxyphényl)-2-méthyl-2-(1-méthylcyclohexyl)propanamide ;
N-(4-méthoxyphénéthyl)-2-méthyl-2-(1-méthylcyclohexyl)propanamide ;
2-méthyl-2-(1-méthylcyclohexyl)-N-(6-méthylpyridin-3-yl)propanamide ;
N-(6-méthoxypyridin-3-yl)-2-méthyl-2-(1-méthylcyclohexyl)propanamide ; et
un mélange correspondant.

3. Composition d'arôme comprenant une quantité acceptable du point de vue olfactif d'un composé selon la revendication 1 ou 2.

4. Composition d'arôme selon la revendication 3 comprenant une quantité acceptable du point de vue olfactif de composés correspondants de formule A et formule B : 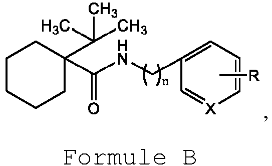
dans laquelle n représente un entier de 0-4 ;
R représente un substituant à une position quelconque du cycle benzénique et est indépendamment choisi dans le groupe constitué par hydrogène, un groupe alkyle en C₁-C₄ et -OR', où R' est un groupe alkyle en C₁-C₄ ; et
X représente C ou N.

5. Composition d'arôme selon la revendication 4, dans laquelle les composés correspondants de formule A et de formule B sont choisis dans le groupe constitué par :
N-(3,4-diméthylphényl)-2-méthyl-2-(1-méthylcyclohexyl)propanamide et 1-(tert-butyl)-N-(3,4-diméthylphényl)cyclohexane-1-carboxamide ;
2-méthyl-2-(1-méthylcyclohexyl)-N-(3,4,5-triméthylphényl)propanamide et 1-(tert-butyl)-N-(3,4,5-triméthylphényl)cyclohexane-1-carboxamide ;
2-méthyl-2-(1-méthylcyclohexyl)-N-phénéthylpropanamide et 1-(tert-butyl)-N-phénéthylcyclohexane-1-carboxamide ;
N-(4-méthoxyphényl)-2-méthyl-2-(1-méthylcyclohexyl)propanamide et 1-(tert-butyl)-N-(4-méthoxyphényl)cyclohexane-1-carboxamide ;
N-(4-éthoxyphényl)-2-méthyl-2-(1-méthylcyclohexyl)propanamide et 1-(tert-butyl)-N-(4-éthoxyphényl)cyclohexane-1-carboxamide ;
N-(4-isopropoxyphényl)-2-méthyl-2-(1-méthylcyclohexyl)propanamide et 1-(tert-butyl)-N-(4-isopropoxyphényl)cyclohexane-1-carboxamide ;
N-(2,4-diméthoxyphényl)-2-méthyl-2-(1-méthylcyclohexyl)propanamide et 1-(tert-butyl)-N-(2,4-diméthoxyphényl)cyclohexane-1-carboxamide ;
N-(4-méthoxyphénéthyl)-2-méthyl-2-(1-méthylcyclohexyl)propanamide et 1-(tert-butyl)-N-(4-méthoxyphénéthyl)cyclohexane-1-carboxamide ;
2-méthyl-2-(1-méthylcyclohexyl)-N-(6-méthylpyridin-3-yl)propanamide et 1-(tert-Butyl)-N-(6-méthylpyridin-3-yl)cyclohexane-1-carboxamide ; et
N-(6-méthoxypyridin-3-yl)-2-méthyl-2-(1-méthylcyclohexyl)propanamide et 1-(tert-butyl)-N-(6-méthoxypyridin-3-yl)cyclohexane-1-carboxamide.

6. Composition d'arôme selon la revendication 5, dans laquelle les composés correspondants de formule A et formule B sont N-(4-méthoxyphényl)-2-méthyl-2-(1-méthylcyclohexyl)propanamide et 1-(tert-butyl)-N-(4-méthoxyphényl)cyclohexane-1-carboxamide.

7. Composition d'arôme selon la revendication 4, dans laquelle la quantité acceptable du point de vue olfactif est d'environ 1 ppb ou plus, et dans laquelle le composé de formule A est présent en une quantité d'environ 1 à 50 % en poids et le composé de formule B est présent en une quantité d'environ 50 % à environ 99 % en poids.

8. Composition d'arôme selon la revendication 3 ou 4, comprenant en outre un composé umami.

9. Composition d'arôme selon la revendication 8, dans laquelle le composé umami est choisi dans le groupe constitué par glutamate monosodique ; 5'-ribonucléotide sodique ; et un mélange de ceux-ci.

10. Procédé pour fournir ou augmenter un goût umami d'une première composition d'arôme comprenant l'étape d'ajout à la première composition d'arôme d'une seconde composition d'arôme, dans lequel la seconde composition d'arôme est une composition d'arôme selon l'une quelconque des revendications 3 à 9.

11. Produit d'arôme comprenant la composition d'arôme selon l'une quelconque des revendications 3 à 9.

12. Utilisation d'un composé selon la revendication 1 ou 2 pour fournir ou augmenter un goût umami d'une composition d'arôme.

13. Utilisation d'un composé selon la revendication 1 ou 2 pour améliorer ou augmenter les caractéristiques aromatiques d'une composition d'arôme.

14. Utilisation d'un composé selon la revendication 1 ou 2 ou d'une composition d'arôme selon l'une quelconque des revendications 3 à 9 dans un produit d'arôme.
